# EUROPEAN PATENT APPLICATION

(11) **EP 4 610 236 A1**
(43) Date of publication of application: **03.09.2025**
(21) Application number: 24160159.0
(22) Date of filing: 28.02.2024
(51) Int. Cl.: C02F 3/34, C12N 1/16, C12N 9/02, C02F 1/28, C02F 1/44, C02F 1/66, C02F 101/34, C02F 103/36, C12R 1/78, C12R 1/84, C12R 1/865

(54) **YEASTS FOR THE TREATMENT OF SALINE WASTEWATER COMPRISING FORMATE**

(71) Applicant: Covestro Deutschland AG, 51373 Leverkusen (DE)
(72) Inventor: The designation of the inventor has not yet been filed
(74) Representative: Levpat

(57) **Abstract**

The present invention relates to means and methods for the biological oxidation of formic acid in industrial wastewater, particularly with high salt concentrations. Yeast cells suitable for this purpose are described as well as useful enzymes, particularly formate dehydrogenases and oxidases.

## Description

The present invention relates to means and methods for the biological oxidation of formic acid in industrial wastewater, particularly with high salt concentrations. Yeast cells suitable for this purpose are described as well as useful enzymes, particularly formate dehydrogenases and oxidases.

4,4'-Methylenedianiline, 2,4'-Methylenedianiline and 2,2'-Methylenedianiline (herein collectively referred to as MDA) are important intermediates in the chemical industry. They are used, e.g. as curing agents in resins or as intermediates in the synthesis of synthesis of methylene diphenyl diicocyanate (MDI). They are obtained by reacting one molecule formaldehyde with two molecules of aniline. The wastewater resulting from this process has a concentration of sodium chloride and is rich in organic contaminants such as formate, phenol, aniline, nitrobenzene and MDA. Formate is an inevitable impurity in the formaldehyde and is carried over into the wastewater resulting from this process.

Moreover, the wastewater resulting from MDA-production have a high salt content. This is also true for organically contaminated wastewaters from many other chemical processes. In the context of the chemical industry it would be desirable to use wastewater with a high salt concentration as raw material. One important process utilizing aqueous solutions having a high sodium chloride content is sodium chloride electrolysis for the production of chlorine, a valuable raw material in its own right. The recycling of high-salt wastewater in this process could create a closed loop.

Unfortunately, sodium chloride electrolysis is highly sensitive to any kind of organic contamination of the brine. Therefore, the wastewater resulting from the synthesis of MDA cannot be used immediately for this purpose without decreasing the content of total organic carbon (TOC). Contaminants such as phenol, aniline or MDA may be removed by adsorption to activated carbon. The removal of formate, however, cannot be achieved by this method.

The Prior Art describes physico-chemical methods for the removal of formate from wastewater. EP 2 096 102 teaches the lowering of the pH of the water, striping with steam and treatment with activated carbon. However, this method does not achieve the required degree purity. WO 2019/158463 describes the removal of formate from saline wastewater by oxidation with ozone and subsequent recrystallization of the salt. This method consumes much energy and is, thus, not suitable for both economic and ecological reasons. Allegedly, the method described in WO 2009/153122 achieves a TOC (total organic carbon) below 25 ppm. This method involves extraction with an organic solvent, steam stripping, use of activated carbon and chemical oxidation using peroxides, ozone or hypochlorites. Obviously, this is a complex and energy-intensive process.

In order to create an economically feasible and ecologically acceptable process for the re-use of wastewater containing formate and high concentrations of sodium chloride, an new process had to be found. This problem is solved by the embodiments of the present invention defined by the claims and in the description below.

In a first embodiment, the present invention relates to a method comprising the steps of
a) providing wastewater containing formic acid;
b) adding a plurality cells of a yeast capable of oxidizing formic acid to said wastewater;
c) incubating the mixture obtained in method step b) under conditions which facilitate formate oxidation of said yeast cells.

In the study underlying the present invention it has been surprisingly found that yeast can be used for the removal of formate from wastewater even if it additionally comprises high concentrations of salt, particularly sodium chloride.

The term "providing" refers to any activity which supplies a wastewater with properties defined by this application in a form that can be used in the process of the invention.

### Wastewater

The actual source of the "wastewater" used in the method of the present invention does not matter provided that it is suitable for the process of the present invention. In particular, wastewater is suitable for this purpose if it meets the specifications set forth below. Wastewater meeting these specifications is preferably the result of a manufacturing process, more preferably a manufacturing process in the chemical industry, even more preferably a chemical synthesis and most preferably the synthesis of MDA from formaldehyde and aniline.

In a preferred embodiment the formate concentration of the wastewater provided in method step a) is between 0.65 and 13.0 mM more preferably between 1.0 and 8.5 mM. It is preferred that the wastewater has a pH of not more than 6. Preferable, however, the pH of the wastewater as provided is at least 3. If necessary, the pH has to be adjusted to meet the limitations defined above. Preferably HCl or NaOH are used for this purpose because the use of other acids or bases introduces ins which interfere with the subsequent use of the purified wastewater. The person skilled in the art is aware that - depending on the pH - there is always an equilibrium between formate (the anion of formic acid) and formic acid as neutral molecule. In the context of the present invention the protonation state of formic acid does not matter. Hence, the terms "formate" and "formic acid" are used interchangeably to designate both free formic acid (neutral) in its anion (deprotonated).

This especially pertains to any concentrations of "formate" or "formic acid" indicated in this application.

As the method of present invention is particularly suited for the treatment of wastewater with a high salt content, in a preferred embodiment of the present invention the concentration of sodium chloride is between 3 wt.-% and 20 wt.-%, preferably 5 wt.-% and 15 wt.-% based on the total mass of all constituents of the wastewater.

In another preferred embodiment of the present invention, the wastewater additionally comprises at least one organic compound selected from the group consisting of phenol, aniline, nitrobenzene and MDA. Phenol, if present, preferably has a concentration between 0.5 and 30.0 mg/l. MDA, if present, preferably has a concentration between 0.01 and 0.5 mg/l. Aniline, if present, preferably has concentration between 0.5 and 15.0 mg/l. Nitrobenzene may be present in traces which may be detected qualitatively but not quantitatively.

### Yeast capable of oxidizing formic acid

A "yeast cell capable of oxidizing formate" is a yeast cell comprising at least one enzyme which catalyzes the oxidation of formate to carbon dioxide. For the method of the present invention it does not matter if the enzyme is native to the cell or if it is recombinantly expressed.

In one preferred embodiment, the yeast cell capable of oxidizing formate expresses a native enzyme catalyzing the aforementioned reaction. In a more preferred embodiment, the expression of the native enzyme is increased. This may be achieved by the introduction of multiple copies of the gene encoding said enzyme under control of a suitable promotor into the cell. It may also achieved by substituting the original promotor sequence of the native gene by a stronger promotor sequence. Any other method known to the Person Skilled in Art for increasing protein expression may also be used.

In another preferred embodiment, the yeast cell capable of oxidizing formate recombinantly expresses an exogenous enzyme catalyzing the aforementioned reaction. All methods known in the art of genetic engineering may be used to achieve that. Exemplary methods are disclosed in the examples section. It is also preferred to express a suitable enzyme recombinantly in a yeast cell already having a native enzyme with the desired enzymic capability.

In a preferred embodiment of the present invention, the yeast cells to be added in method step b) are precultured in a medium containing 0.1 to 10.0 vol.-%, preferably 0.2 to 3.0 vol.-% and most preferably 0.3 to 2.0 vol.-% methanol. It has been shown that such a pre-incubation increases the activity of the relevant enzymes.

### Formate dehydrogenase

The term "format dehydrogenase" is known to the person skilled in the art. It refers to any enzyme having the capability to transfer electrons from formate to a coenzyme, preferably NAD⁺ and thereby oxidizing formate to carbon dioxide. Preferably, a "formate dehydrogenase" as referred to in the present application belongs to EC class 1.17.1.9.

Preferred formate dehydrogenases are the enzymes from *Candida boidinii* and *Hansenula polymorpha.* Preferably, said enzyme as an amino acid sequence as defined by SEQ ID NO.: 1 or 2 or an amino acid sequence having at least 90 %, preferably at least 95 % sequence identity with SEQ ID NO.: 1 or 2, provided that an enzyme with such sequence identity still has at least 20 %, preferably at least 50 % of formate dehydrogenase activity of the original enzyme as defined by SEQ ID NO.: 1 or 2. This can be tested using commonly known enzyme assays.

### Formate Oxidase

The term "formate oxidase" is known to the person skilled in the art. It refers to any enzyme having the capability to tranfer electrons from formate to molecular oxygen and thereby oxidizing formate to carbon dioxide. Preferably, a "formate oxidase" as referred to in the present application belongs to EC class 1.2.3.1.

Preferred formate oxidases are the enzymes derived from *Schwanniomyces vanrijiae* or *Aspergillus oryzae.* Preferably, they have amino acid sequences as defined by SEQ ID NO 3 or SEQ ID NO.: 4 or an amino acid sequence having at least 90 %, preferably at least 95 %, sequence identity with one of the aforementioned sequences, provided that an enzyme with such sequence identity still has at least 20 %, preferably at least 50 % of formate oxidase activity of the original enzyme as defined by SEQ ID NO.: 3 or 4. This can be tested using commonly known enzyme assays

Further preferred variants of the above-described enzymes are those that are derived from SEQ ID NO.: 1, 2, 3 or 4 by addition, deletion or substitution of up to 20, preferably up to 15, more preferably up to 10 and most preferably up to 5 amino acids. In principle, said additions, deletions or substitutions can be made at any part of said sequences in a continuous or discontinuous fashion. It is, however, preferred to make such changes at the amino terminus and/or the carboxy terminus of the polypeptide. It is a requirement that any such variant retains formate oxidase activity or formate dehydrogenase activity as defined above.

### Incubating

The term "incubating" in method step c) refers to the act of contacting the yeast cells and the wastewater under conditions which facilitate the oxidation of formate by the above-defined enzymes or any native formate dehydrogenase or formate oxidase or any other native enzyme system comprised by the yeast cells. Generally, the person skilled in the art knows the conditions which are required based on the known temperature and pH limits of enzymatic activity. In the context of the present invention it is not required that the yeast cells remain viable, i.e. capable of proliferation, as they are only intended to serve as carriers for the enzymes. However, the conditions under which incubation is performed preferably maintain integrity of the cell membrane. If the yeast cell comprises a formate dehydrogenase, it is additionally preferred that the respiration chain remains functional under the conditions selected for incubation. As shown in the study underlying the present invention these requirements are met if wastewater with the composition defined further above in the specification is used and incubation takes place at temperatures between 5 °C and 50 °C, preferably between 5 °C and 35 °C. Incubation preferably takes place at a pH between 3.5 and 6, more preferably between 4. And 5.5.

It is preferred to continue incubation until at least 90 %, more preferably at least 95 % of the formate originally comprises by the wastewater are consumed. In a more preferred embodiment of the present invention method step c) is continued until formate concentration in the wastewater does not exceed 0.2 mM, preferably 0.1 mM and most preferably 0.02 mM.

### Recycling of yeast cells

In a preferred embodiment of the present invention the yeast cells are separated from the purified wastewater at the end of method step c). In one embodiment this may be achieved by filtration. This may be achieved by all filtration methods known in the art of biotechnology, preferably microfiltration or ultrafiltration. Preferably, the separated cells are then added to a new batch wastewater (equivalent to method step b)). Thus, a recycling of the yeast cells may be achieved, thus increasing the efficiency of the process. It is preferred to reuse the yeast cells at least 10, preferably at least 25 and most preferably at least 40 times as it has been shown that the activity does not deteriorate to a relevant degree.

### Use of purified wastewater

In a preferred embodiment of the present invention the purified wastewater resulting from method step c) is used in the chloralkali process for the generation of chlorine. This use is well known in the art. If such a use of the wastewater is intended, it is preferred to remove the cells at the end of methods step from the purified wastewater because any organic contaminant may negatively affect the electrolysis process. This can be achieved by any suitable method, particularly microfiltration or ultrafiltration as defined above. However, filtration using activated carbon is another preferred method as this method also removes many other organic contaminants which may be present in the wastewater, particularly phenol, aniline, nitrobenzene and MDA.

In another embodiment, the present invention relates to the use of a yeast cell
a) expressing a formate dehydrogenase or formate oxidase as native enzyme or
b) recombinantly expresses at least one formate dehydrogenase or formate oxidase for removing formic acid from wastewater.

In yet another embodiment, the present invention relates to the use of a formate dehydrogenase or formate oxidase recombinantly expressed in a yeast cell for the degradation of formic acid.

### Advantages

The process of the present invention is simple, consumes little energy and produces water with a very low residual amount of formate that can be either safely released into the environment or advantageously used as raw material for sodium chloride electrolysis. It does not involve chemicals that may be harmful, expensive or require large amounts of energy in the production. It also does not require a high energy input such as steam stripping.

The following examples are merely intended to illustrate the present invention. They shall not limit the scope of the claims in any way.

### Examples

### Material & methods:

### 1. Preparation of integration plasmids for yeast transformation

The methylotrophic yeasts *P. pastoris* (X-33) and *H. polymorpha* (DSM 70277) were transformed by using common integration plasmids (*P. pastoris:* pPICZA, *H. polymorpha:* pHIPH4). The respective genes coding formate oxidases were isolated via colony PCR from *Schwanniomyces vanrijiae* (DSM 70252, amino acid sequence of the enzyme as defined by SEQ ID NO.: 3) and *Aspergillus oryzae* (DSM 63303, amino acid of the enzyme as denied by SEQ ID NO.: 4). The CbFDH-gene was amplified from a pET21a-CbFDH plasmid (amino acid sequence of the enzyme as defined by SEQ ID NO.: 1). Gibson assembly was used for integration of the respective genes into the plasmids by using NEBuilder^{®} HiFi DNA Assembly Master Mix (New England BioLabs^{®} Inc.). 2 µL of the reaction mixture was used to transform chemically competent *Escherichia coli* DH5α cells.

After verification of correct plasmid construction by sequencing, one colony of each plasmid construct was selected and cultivated overnight in LB low salt medium (10 g/L tryptone, 5 g/L yeast extract, 5 g/L NaCl, pH=7.5) with 25 µg/mL zeocin (pPICZA) or 100 µg/mL ampicillin (for pHIPH4) at 37 °C, 200 rpm. Plasmid preparation was used to obtain plasmids for yeast transformation. Before the transformation, plasmids were linearized within the promoter region (or before the promoter, table 1) and afterwards cleaned with a PCR purification kit. 1-10 µg of linearized plasmid-DNA were used for each transformation via electroporation.

**Table 1: Used restriction enzymes for the linearization of each plasmid.**

| **Plasmid** | **Restriction enzyme** |
|---|---|
| pHIPH4 | FastDigest^{™} Notl |
| pHIPH4-AoFOx | FastDigest^{™} Eco147l |
| pHIPH4-CbFDH | FastDigest^{™} Notl |
| pHIP4-DvFOx | FastDigest^{™} Pael |
| pPICZa | FastDigest^{™} Oral |
| pPICZA-AoFOx | FastDigest^{™} Oral |
| pPICZA-CbFOH | FastDigest^{™} Oral |
| pPICZA-DvFOx | FastDigest^{™} Sacl |

### 2. Generation of electrocompetent yeast cells and transformation

Cryogenic cultures of the wildtype *P. pastoris* and *H. polymorpha* strains were plated out on YPD agar plates (10 g/L yeast extract, 20 g/L peptone, 10 g/L glucose, 20 g/L agar) containing 100 µg/mL ampicillin and incubated at 30 °C. One colony was picked and cultivated overnight in YPD medium with 100 µg/mL ampicillin overnight at 30 °C in a cultivation tube. A shake flask containing 50 mL YPD medium with 100 µg/mL ampicillin is inoculated with 50 µL preculture and cultivated at 200 rpm, 30 °C. After reaching the target OD₆₀₀, cells were harvested and washed with the respective solutions according to table 2. The used STM buffer was prepared by mixing 1 % (v/v) of 1 M TRIS solution (pH=7.5) with 0.1 % (v/v) of a 1 M MgCl₂ solution and 80 % of the final volume of deionized water. 92.4 g/L D(+)-sucrose was added and after full dissolution the buffer is filled up to the final volume with deionized water.

**Table 2: Cell specific procedure for generation of electrocompetent H. polymorpha and P. pastoris cells. All wash steps are normalized for 50 mL main culture.**

| **Protocol step** | ***H. polymorpha*** | ***P. pastoris*** |
|---|---|---|
| Medium | YPD | YPD |
| OD₆₀₀ main culture | 0.8-1.2 | 1.3-1.5 |
| Centrifugation steps | 700x g, 10 min, 20 °C | 1500x g, 5 min, 4 °C |
| 1. wash | 10 mL 50 mM KPi buffer (pH=7.5, 37 °C) + 250 µL 1 M DTT incubated for 20 min at 37 °C | 50 mL ice-cold water |
| 2. wash | 50 mL STM buffer | 25 mL ice-cold water |
| 3. wash | 25 mL STM buffer | 2 mL ice-cold 1 M sorbitol |
| Final resuspension, aliquots | 250 µL STM buffer, 80 µL aliquots | 100 µL 1 M sorbitol, 80 µL aliquots |

For transformation of the methylotrophic yeasts, always freshly prepared aliquots of competent cells were used. 1 µg up to 10 µg of linearized plasmid-DNA is pipetted into the aliquots. The complete mixture is transferred to an electroporation cuvette. Cell specific electroporation parameters (voltage, capacity, number of pulses etc.) are summarized in table 3.

After transformation and regeneration, all transformation mixtures are centrifugated (20 °C, 700x g, 5 min). 900 µL of the supernatant are discarded. The cell pellet is resuspended in the remaining medium (^{~} 100 µL) and is streaked out on a selective plate (pPICZA: 100 µg/mL ampicillin + 100 µg/mL zeocin; pHIPH4: 100 µg/mL ampicillin + 300 µg/mL hygromycin). The plates are incubated for up to 3 days at 30 °C or 37 °C. For methylotrophic yeasts, at most 48 colonies per construct representing 48 transformants are picked and used for inoculation of a sterile 96-well master plate with 200 µL YPD medium per well (+ 100 µg mL⁻¹ ampicillin and the respective eukaryotic antibiotics). After at most 3 days, 50 µL 87 % (v/v) glycerol is added and the master plate is stored at -80 °C until flower plate expression cultivation and screening via indicator assay.

**Table 3: Transformation of H. polymorpha and P. pastoris.**

| **Parameter** | ***H. polymorpha*** | ***P. pastoris*** |
|---|---|---|
| Plasmid-DNA | ≤ 10 µg linearized plasmid-DNA | ≤ 10 µg linearized plasmid-DNA |
| Voltage | 2 kV | 1.5 kV |
| Number of pulses | 1 | 1 |
| Capacity | 25 µF | 25 µF |
| Resistance | 200 Ω | 200 Ω |
| Cuvette | 2 mm | 2 mm |
| Regeneration | 1 mL YPD, 1 h, 37 °C, 800 rpm (thermomixer) | 1 mL 1 M sorbitol, 1 h , 30 °C, 0 rpm |
| Selection | YPD plates + 100 µg/mL amp + 300 µg/m L hyg | YPD plates + 100 µg/mL amp + 100 µg/mL zeo |

### 3. Expression protocols

For protein expression in methylotrophic yeasts, different media were used during the cultivation process. The media were summarized in table 4.

**Table 4: Media composition for YPD, BMGY, BMMY and BMMH medium. The media were prepared freshly by mixing the respective volums of a 10/7x YP stock solution (yeast extract, peptone, with and without NaCl), 20x glucose solution, pure methanol, 5000x biotin solution, 10x KPi buffer stock solution and 10x Yeast Nitrogen Base (YNB, without amino acids) stock solution. NaCl was only added for salt adaption with the selected clones.**

| **Component** | **YPD** | **BMGY** | **BMMY** | **BMMH** |
|---|---|---|---|---|
| Yeast extract [g L⁻¹] | 10 | 10 | 10 | - |
| Peptone [g L⁻¹] | 20 | 20 | 20 | |
| Glucose [g L⁻¹] | 10 | 10 | | |
| Methanol [% (v/v)] | - | - | 1 | 0.5 |
| Biotin [mg L⁻¹] | | 0.4 | 0.4 | 0.4 |
| KPi buffer, pH=6 [M] | - | 0.1 | 0.1 | 0.1 |
| YNB [g L⁻¹] | - | 13.4 | 13.4 | 13.4 |
| NaCl [g L⁻¹] | - | 40 | 40 | 40 |

A 3 mL YPD preculture (with 100 µg/mL ampicillin and the respective eucaryotic antibiotic) was inoculated from a cryogenic culture or with one positive selected well from the master plate. The preculture was conducted at 30 °C and 200 rpm overnight. OD₆₀₀ was measured. A 1 L shake flask containing 25 mL BMYG (*H. polymorpha*) or BMMH medium with 10 g L⁻¹ glucose instead of methanol (*P. pastoris*) and the respective selection reagent was inoculated with OD₆₀₀=0.1. This main cultured was cultivated at 200 rpm and 30 °C for 48 hours and was salt adapted. After that, the same volume as started of BMMY with 2 % methanol (*H. polymorpha*) or BMMH with 1 % methanol (*P. pastoris*) was added. After additional 24 h of cultivation, 1/10 of the total liquid volume in the flask of BMMY with 10 % methanol (*H. polymorpha*) or BMMH with 5 % methanol (*P. pastoris*) was added to the flask. The cultivation is continued for 24 h. Cells were harvested by centrifugation at 8000xg for 10 min. The supernatant is discarded and the cells were used for formate degrading experiments and sodium dodecyl sulfate polyacrylamide gel electrophoresis (SDS-PAGE) or stored at -20 °C until use.

For identification of the best variant of the yeasts with each construct, the expression protocol was conducted as described before in a 48-well flower plate without additional salt in the medium. 800 µL medium was used as start for biomass generation. After 48 h, 800 µL BMMY (2% methanol) or BMMH (1 % methanol) was added. After additional 24 h, 160 µL BMMY (10 % methanol) or BMMH (5 % methanol) was supplemented. The indicator assay was conducted after 96 h total cultivation time to identify clones with high protein expression. Afterwards, the flower plate was stored at - 20 °C until SDS-PAGE is conducted for the best transformants. These transformants were used from the master plate for further cultivations and degradation experiments.

### 4. Indicator assay for identification of clones with high protein expression

For identification of the best yeast variants, a pH indicator assay was established during this study. As standard assay, 200 µL per MTP well are used. Cultivated cells were diluted to OD₆₀₀=0.5 in the assay. Therefore, the OD₆₀₀ of three wells after a 48-well flower plate cultivation were measured. The mean value of these measurements was used to calculate the dilution of the cell suspensions. Furthermore, the OD₆₀₀ of each well is measured in a 1:10 dilution in a 96-well MTP. 1x PBS is used for dilution.

For the assay, each well contains 1.7 µL bromcresol purple solution and 8.5 mM sodium formate. 25 mM KPi bufffer (pH=5.6) is used as medium and for dilution steps. The assay was conducted in a photometer at 25 °C for up to 5h at 600 rpm and absorbance is measured at 598 nm (deprotonated indicator species, table 5). The linear increase in absorbance over time was used for the activity of the different transformants. The slopes are normalized with the previous measured OD₆₀₀ for each transformant. The transformants with highest normalized slopes were checked for protein expression via SDS-Page electrophoresis and were used for further analysis.

**Table 5: pH Indicator assay parameter and conditions.**

| **Parameter** | **Condition** |
|---|---|
| Temperature | 25 °C |
| Wavelength | 598 nm |
| Measurement time | > 5 h |
| Well volume | 200 µL |
| Buffer | 25 mM KPi, pH=5.6 |
| OD₆₀₀ | 0.5 |
| Bromcresol purple | 0.05 mM |
| Sodium formate | 8.5 mM |

### 5. Formate degradation experiments

Small scale experiments regarding the activity of cells to degrade formate were conducted in 15 mL round well tubes at 200 rpm in an incubator shaker at different temperatures. OD₆₀₀=5 normalized cell pellets were resuspended in 1.98 mL of Mcllvaine buffer with 10 % (w/v) NaCl and the respective pH value. Degradation experiments were started by adding 14 µL of a 1 M sodium formate solution. 150 µL samples were taken at different times and are heat inactivated at 95 °C for 5 min. Afterwards, the samples were centrifugated (5 min, 13300 rpm). The supernatant was transferred to a new tube and stored at 4 °C until formate quantification by FDH-assay.

For formate quantification, an FDH-assay was used (table 6). 200 µL per MTP well were used and the absorbance at 340 nm is measured at 37 °C for 3 h in a photometer. The last measurement was used for formate quantification. A calibration curve was measured by measuring triplicates of varying formate concentrations between 5 µM and 0.4 mM. As blank for standards and samples the CbFDH enzyme was replaced by KPi buffer. Blanks were measured at least as duplicates and samples as triplicates. Sample volume was varied to dilute the formate concentration into the standard range.

**Table 6: FDH assay parameter and conditions.**

| **Parameter** | **Condition** |
|---|---|
| Temperature | 37 °C |
| Wavelength | 340 nm |
| Measurement time | > 3 h |
| Well volume | 200 µL |
| Buffer | 50 mM KPi, pH=7.6 |
| 50 mM NAD⁺ | 4 µL (1mM in excess) |
| 4 U mL⁻¹ CbFDH | 50 µL (1 U mL⁻¹) |
| Sodium formate range | 5 µM - 0.4 mM |
| Standard/samples | Triplicates |
| Blanks | Duplicates |

Examination of cell stability was conducted in DASbox^{®} mini bioreactors. A script was implemented in the software to simulate the preferred mode of operation. The formate degradation was tracked by the volume of pumped 0.28 M HCl solution with 100 g/L NaCl to hold the pH value constant. After 95 % of the theoretically necessary volume of HCl solution was consumed, the next cycle was started by automatically adding 1 mL 0.7 M sodium formate solution with 100 g/L NaCl.

Before the experiments were started, pump calibration as well as DO (Dissolved oxygen) probe and pH probe calibration are conducted according to the DASbox^{®} control software. The reactors were filled with 90 mL 10 % (w/v) NaCl containing water. OD₆₀₀=5 normalized cell pellets corresponding to 100 mL reaction volume are resuspended in 10 mL 10 % (w/v) NaCl containing water. This suspension is pumped into the reactor by using a feed channel and a 15 mL syringe. Afterwards, the pH value was adjusted with the 0.28 M HCl solution or externally with a 1 M NaOH solution. The script was started by pumping the first 1 mL of 0.7 M sodium formate solution in the reactor.

### Results:

### 1. Tests of commercially available baker's yeasts

Baker's yeasts of six different suppliers were also tested for their capability for formate degradation. OD₆₀₀ was normalized to approximately 10 (20 mg mL⁻¹) and suspended in 10 % (w/v) NaCl containing buffer. Formate concentration was quantified at the beginning of the degradation experiment and after 24 h (table 7).

**Table 7: Measured formate concentrations for commercially available baker's yeasts in buffered system with 10 % NaCl after 24 h. OD₆₀₀=10, T=30 °C, c(t=0 h)=6.43±0.20 mM, pH=5.0.**

| **Baker's yeast supplier/brand** | **C_{Formate} [mM]** |
|---|---|
| Rewe Bio, REWE-ZENTRALFINANZ eG, Köln | 5.51 |
| Agrano GmbH & Co. KG, Riegel | 5.72 |
| Lesaffre Deutschland GmbH, Kehl | 6.72 |
| Dr. August Oetker Nahrungsmittel KG, Bielefeld | 1.12 |
| Deutsche Hefewerke GmbH, Nürnberg | 6.27 |
| UNIFERM GmbH & Co. KG, Werne | 0.01 |

### 2. Identification of high expressing yeast transformants

After conducting the described transformation and screening procedure, SDS-PAGEs of the best performing *H. polymorpha* were prepared to identify transformants with high expression of the respective proteins.

For the proteins AoFOx and DvFOx, transformants with a good protein expression were identified, because of their subunit molecular mass of 64 kDa. An overexpression of the CbFDH protein was not observed on the SDS-PAGE. The transformants C1 (AoFOx), D8 (DvFOx) and E6 were used for further analyses.

The same procedure was conducted to identify *P. pastoris* transformants with high expression of the respective proteins.

For the AoFOx and DvFOx transformants, protein expression was observed. Protein overexpression was not observed for the CbFDH transformants. The transformants B7 (AoFOx), B7 (DvFOx) and B2 (CbFDH) were selected for further analyzes.

### 3. pH optimization

The selected strains were cultivated in shake flask scale and evaluated regarding their pH dependent activity in buffered systems with 10% NaCl (table 8).

**Table 8: Measured formate concentrations for all H. polymorpha and P. pastoris strains dependent of the pH in the buffered system after 4 h. OD₆₀₀=5, T=25 °C, c(t=0 h)=6.3±0.9 mM, * = measured after 3 h.**

| | | **pH [-]** | | | | |
|---|---|---|---|---|---|---|
| **Organism** | **Strain** | **4.0** | **4.5** | **5** | **5.5** | **6** |
| *H. polymorpha* | Wildtype | 3.2 mM | 3.3 mM | 3.2 mM | 4.1 mM | 5.2 mM |
| | AoFOx^{∗} | 1.7 mM^{∗} | 2.1 mM^{∗} | 2.4 mM^{∗} | 4.2 mM^{∗} | 5.4 mM^{∗} |
| | CbFDH | 5.8 mM | 4.4 mM | 4.5 mM | 4.7 mM | 4.8 mM |
| | DvFOx | 0 mM | 0 mM | 0.9 mM | 3.0 mM | 4.5 mM |
| *P. pastoris* | Wildtype | 0.5 mM | 0.6 mM | 1.5 mM | 2.7 mM | 3.9 mM |
| | AoFOx | 0 mM | 0 mM | 0 mM | 1.4 mM | 3.1 mM |
| | CbFDH | 0 mM | 0 mM | 0 mM | 0.4 mM | 2.1 mM |
| | DvFOx | 0 mM | 0 mM | 0 mM | 0.3 mM | 1.3 mM |

For all transgenic strains higher activity was observed compared to the respective wildtypes except the *H. polymorpha* CbFDH transformant. This indicated the soluble and active enzyme production in these transformants. For stability evaluation experiments, pH was set to 5.0 for the FOx and to 5.5 for the other strains to avoid too acidic stress to the stainless-steel bioreactor.

### 4. Stability evaluation

The catalytic performance of all selected transformants was evaluated in bioreactor experiments and compared to the respective wildtype for the yeasts. As explained in previous sections, the bioreactor was used for repeated batch formate degradation to simulate the cell recycling of the real application in an unbuffered system. A formate supplementation was triggered by an internal script if 95 % of theoretical needed acid volume was pumped into the reactor. The consumed volume of HCl solution to hold the pH value during formate degradation constant was used as indication of the catalytic performance of the cells during bioreactor runs. The mean duration of the first three runs, the duration of the last run and the duration of active formate degradation were summarized as indicators for activity and stability. As the reactor runs were not conducted for the same time frame, the first 96 h were defined as time frame for comparison between the transformants and the yeasts. The mean time per cycle in 96 h was used as KPI for comparison. Only completed cycles were considered for the calculation.

For the FOx transformants of both yeasts, formate degradation was conducted at pH=5.0 whereas the experiments for the wildtypes and the CbFDH transformants were conducted at pH=5.5. OD₆₀₀ of all strains was normalized to 5 in 100 mL. The temperature was set to 25 °C. The KPIs of the *H*. *polymorpha* strains were summarized in table 9 and the KPIs of the *P. pastoris* strains were summarized in table 10.

**Table 9: Summary of the KPIs as well as the improvement factors of the H. polymorpha strains compared to the wildtype during the formate degradation in the bioreactor.**

| **Strain** | **Wildtype** | **AoFOx** | **CbFDH** | **DvFOx** |
|---|---|---|---|---|
| **Mean duration of cycle 1-3 [h]** | 6.6 | 2.2 | 6.8 | 3.7 |
| **Duration last cycle [h]** | 18.7 | 17.5 | 28.7 | 31.8 |
| **Active duration [h]** | >137 | 83 | 90 | 90 |
| **Mean duration per cycle in 96 h [h cycle⁻¹]** | 8 | 5.1 | 12 | 8 |
| **Improvement factor [-]** | 1 | 1.6 | 0.7 | 1 |

**Table 10: Summary of the KPIs as well as the improvement factors of the P. pastoris strains compared to the wildtype during the formate degradation in the bioreactor.**

| **Strain** | **Wildtype** | **AoFOx** | **CbFDH** | **DvFOx** |
|---|---|---|---|---|
| **Mean duration of cycle 1-3 [h]** | 7.6 | 1.4 | 4.0 | 1.2 |
| **Duration last cycle [h]** | 32.7 | 12.8 | 27.1 | 15.1 |
| **Active duration [h]** | 67 | 112 | 101 | 60 |
| **Mean duration per cycle in 96 h [h cycle⁻¹]** | 24 | 2.3 | 9.6 | 3.6 |
| **Improvement factor [-]** | 1 | 10.4 | 2.5 | 6.7 |

A higher starting activity was observed for all transgenic strains except the *H. polymorpha* CbFDH strain which was in the range of the wildtype. Except of the *H. polymorpha* CbFDH and DvFOx strain, an improved catalytic performance was observed for all transgenic strains. The AoFOx strains of both yeasts showed the highest catalytic performance, which is coherent with their high protein expression observed on the conducted SDS-PAGEs.

As last experiment, the best variants and the wildtypes of both yeasts were tested in real MDA-process wastewater with previous activated carbon treatment and without such a treatment. TOC and formate were quantified before and after treatment by the cells (table 11).

**Table 11: TOC and formate quantification of real MDA-process wastewater (with and without previous activated carbon treatment) before and after treatment with yeast cells. Conducted in 100 mL bioreactors, formate quantified by FDH-assay with 3 analytical replicates. OD₆₀₀=5. Duration up to 10 h. TOC was quantified using the vario TOC cube (Elementar Analysesysteme, Germany)**

| | | **Wastewater with previous activated carbon treatment** | | **Wastewater without previous activated carbon treatment** | |
|---|---|---|---|---|---|
| **Strain** | **Parameter** | **Before cell treatment** | **After cell treatment** | **Before cell treatment** | **After cell treatment** |
| *H. polymorpha* wildtype | Formate in mM | 5.3 | 0.0 | 5.4 | 0.1 |
| | TOC in ppm | 64 | 8.7 | 64 | 13.6 |
| *H. polymorpha* AoFOx | Formate in mM | 5.3 | 0.0 | 5.4 | 0.2 |
| | TOC in ppm | 64 | 11.6 | 64 | 10.3 |
| *P. pastoris* wildtype | Formate in mM | 5.3 | 1.1 | 5.4 | 0.2 |
| | TOC in ppm | 64 | 27 | 64 | 19.1 |
| *P. pastoris* AoFOx | Formate in mM | 5.3 | 0.0 | 5.4 | 0.0 |
| | TOC in ppm | 64 | 15 | 64 | 10.9 |

The results have shown that all strains, but especially the wildtype strains were capable to oxidize the formate in real MDA-process wastewater. The remaining TOC was counted to cell lysis during the treatment. The results were showing the suitability of formate degradation in high saline wastewaters by (transgenic) methylotrophic yeasts.

## Claims

1. A method comprising the steps of
a) providing wastewater containing formic acid;
b) adding a plurality cells of a yeast capable of oxidizing formic acid to said wastewater;
c) incubating the mixture obtained in method step b) under conditions which facilitate formate oxidation of said yeast cells.

2. The method of claim 1, wherein method step c) is continued until at least 90 % of the formate comprised by the wastewater provided in method step a) have been consumed.

3. The method of claim 1 or 2, comprising after method step c) an additional method step d) of separating the cells from the wastewater.

4. The method of claim 3, wherein the separated cells are reused in method step b).

5. The method of claim 3, wherein the cells are separated from the wastewater by filtration with activated carbon.

6. The method of any one of claims 1 to 5, wherein the concentration of formic acid in the wastewater provided in method step a) is between 0.65 and 13 mM.

7. The method of any one of claims 1 to 6, wherein the wastewater provided in method step a) has a pH below 6.

8. The method of any one of claims 1 to7, wherein the wastewater has a concentration of sodium chloride between 3 wt.-% and 30 wt.-% based on the total mass of the wastewater.

9. The method of claim 8, wherein subsequent to method step c) or d) the wastewater is used in the chloralkali process.

10. The method of any one of claims 1 to 9, wherein a yeast is used which expresses a formate dehydrogenase or formate oxidase as native enzyme.

11. The method of any one of claims 1 to 9, wherein the yeast cell recombinantly expresses at least one formate dehydrogenase or formate oxidase.

12. Use of a yeast cell expressing a formate dehydrogenase or formate oxidase as native enzyme
for removing formic acid from wastewater.

13. Use of a formate dehydrogenase or formate oxidase recombinantly expressed in a yeast cell for the degradation of formic acid.
